# EUROPÄISCHE PATENTSCHRIFT

(11) **EP 3 072 463 B1**
(45) Veröffentlichungstag und Bekanntmachung des Hinweises auf die Patenterteilung: **17.08.2022**
(21) Anmeldenummer: 15160874.2
(22) Anmeldetag: 25.03.2015
(51) Int. Cl.: A61B 17/3207

(54) **Medizinisches Kit bzw. Set zur Behandlung eines krankhaft veränderten Hohlorgans**
Medical kit or set for treating a diseased hollow organ
Kit médical ou set pour traiter un organe creux malade

(43) Veröffentlichungstag der Anmeldung: 28.09.2016
(73) Patentinhaber: B. Braun Melsungen AG, 34212 Melsungen (DE); medicut Stent Technology GmbH, 75179 Pforzheim (DE)
(72) Erfinder: Thalwitzer, Jörg, 08066 Zwickau (DE)
(74) Vertreter: Patentanwälte Ruff, Wilhelm, Beier, Dauster & Partner mbB

(56) Entgegenhaltungen:
- WO-A1-98/38926
- WO-A2-2010/034021
- DE-A1-102005 024 930
- DE-B3-102011 120 004
- US-A1- 2005 021 071
- US-A1- 2005 216 050
- US-A1- 2006 259 005
- US-A1- 2007 016 232
- US-A1- 2011 160 645

## Beschreibung

Die Angioplastie, auch als perkutane transluminale Angioplastie (PTA) oder perkutane transluminale Koronarangioplastie (PTCA) bezeichnet, ist ein Verfahren zur Erweiterung oder Wiedereröffnung von verengten oder verschlossenen Blutgefäßen.

Die Angioplastie wird üblicherweise mittels Ballondilatation durchgeführt. Unter einer Ballondilatation im Rahmen einer Angioplastie versteht man in der interventionellen Radiologie, der Kardiologie, Gefäßchirurgie sowie der Angiologie eine Technik zur Aufdehnung krankhaft verengter Blutgefäße mittels eines Ballon-Katheters. Ein Ballon-Katheter ist ein dünner, flexibler Kunststoffschlauch, welcher an einem distalen Schlauchabschnitt einen mittels Flüssigkeit inflatierbaren, d.h. entfaltbaren bzw. aufweitbaren, Ballon (Okklusionsballon) aufweist. Üblicherweise wird der Ballon-Katheter von einem Gefäß im Arm oder Bein aus über einen Führungsdraht oder Führungskatheter in die Engstelle (Stenose) platziert und unter hohem Druck (6 bis 20 bar) entfaltet. Hierdurch wird meist die Engstelle beseitigt und eine Operation vermieden.

Nachteilig bei der oben beschriebenen Ballonangioplastie ist, dass regelmäßig traumatische Dissektionen und Gefäßwandeinblutungen auftreten. Diese können ursächlich für eine Gerinnselbildung und Verschleppung (Embolie) sowie einen erneuten Gefäßverschluss, d.h. eine sogenannte Restenose, sein. Gefäße mit derart unzureichenden PTA-Ergebnissen werden häufig mittels einer Stentimplantation behandelt. Da der Stent jedoch ein Fremdmaterial darstellt, besteht ein gewisses Risiko, dass der Stent seinerseits pathologische Reaktionen verursacht, welche ebenfalls zu einer Restenose oder zu erschwerten OP-Bedingungen bei einem operativen Zweiteingriff führen können. Hinzu kommt, dass ein implantierter Stent einer doppelten Thrombozytenaggregation bedarf und hierdurch ein erhöhtes Blutungsrisiko für den Patienten besteht.

Häufig kommen auch medikamentenbeschichtete Ballonkatheter zum Einsatz. Bei derartigen Kathetern ist der Ballon mit einem besonderen Medikament, wie beispielsweise Paclitaxel, beschichtet. Das Medikament wird während der Dilatation direkt in die Gefäßwandung abgegeben. Dadurch soll das Risiko verringert werden, dass sich Narbengewebe bildet, was eine der häufigsten Ursachen für eine Restenose darstellt.

Problematisch ist jedoch, dass arteriosklerotische Ablagerungen sowie die Bildung von Narbengewebe eine Verdickung der Gefäßwandung auf der Innenseite bewirken, wodurch eine Medikamentendiffusion erschwert oder vollständig verhindert wird.

Bei der sogenannten Schneid-Angioplastie (Blade-Angioplastie) kommen Ballonkatheter mit auf der Ballonaußenfläche angebrachten Schneidelementen zum gezielten Einschneiden von arteriosklerotischen Plaque-Ablagerungen und der Gefäßinnenwand, auch als Schneid-Ballonkatheter bezeichnet, zum Einsatz.

Aus der US 5,320,634 ist es beispielsweise bekannt, einen Ballon eines Ballon-Katheters mit Schneiden auszustatten. Wird der Ballon an den verengten Blutgefäßen dilatiert, schneiden diese Schneiden die arteriosklerotischen Ablagerungen und die Gefäßinnenwand gezielt und gewollt auf. Ein Ballonkatheter mit Schneidflügeln auf der Außenfläche des Ballons ist aus der WO 03/049603 A2 bekannt.

Nachteilig bei den Schneid-Ballonkatheter ist, dass ihre Ballondurchmesser normiert sind, typischerweise in einem Bereich von 2 mm bis 12 mm. Dies hat zur Folge, dass zur Behandlung eines Gefäßes, welches physiologisch bedingt unterschiedliche Innendurchmesser besitzt, mehrere (teure) Ballonkatheter erforderlich sind. Hinzu kommt, dass die kommerziell erhältlichen Schneid-Ballonkatheter ein relativ großes Einführbesteck benötigen, wodurch sich die Handhabung für den behandelnden Mediziner zusätzlich verkompliziert.

Weitere Probleme können beim Entfernen des Schneid-Ballonkatheters aus dem behandelten Gefäß auftreten. Hierbei können nämlich Verdrillungen zwischen Ballon und Schneidelement oder zwischen Schneidelement und einem dilatierten Plaque auftreten, was den Entfernvorgang signifikant erschweren kann.

Andere Lösungen sind aus der DE 10 2005 024 930 A1 sowie US 2005/0080478 A1 bekannt. Die aus der DE 10 2005 024 930 A1 bekannte Vorrichtung weist einen Schneidabschnitt zum Aufbrechen von Ablagerungen in einem Körpergefäß auf. Der in der US 2005/0080478 A1 beschriebene Stent, welcher dauerhaft im Körper verbleiben soll, ist mit Schneidkanten versehen. Der Nachteil dieser Lösung besteht darin, dass eine dauerhafte Reizung des Gefäßabschnitts durch die Schneidkanten entsteht. Dies kann die Ablagerung von arteriosklerotischen Plaques oder eine Restenose begünstigen.

Eine weitere Möglichkeit zur Behandlung von Gefäßverengungen bieten sogenannte Rotablatoren. Dies sind rotierende Schneidköpfe, die eine Stenose auffräsen sollen. Nachteilig ist hierbei eine erhöhte Verletzungsgefahr durch den rotierenden Schneidkopf. Ein zusätzliches Risiko besteht darin, dass zu entfernende Plaque-Ablagerungen in weiter distal gelegene Gefäßabschnitte (Embolie) verstreut werden können, woraus eine verschlimmerte Durchblutungssituation und drastischere chirurgische Konsequenzen resultieren können.

Eine Atherektomie-Vorrichtung mit einem Schneidelement sowie einer hüllenförmigen Einführhilfe für das Schneidelement ist aus der WO 98/38926 A1 bekannt.

Gegenstand der WO 2010/034021 A2 ist ein System zum Entfernen von obstruktivem Material aus einem Körperlumen. Das System weist eine Mazerationsvorrichtung mit einer expandierbaren Käfigstruktur sowie einem Begrenzungsschlauch für die Käfigstruktur auf, wobei der Begrenzungsschlauch dazu eingerichtet ist, die Käfigstruktur in einer kollabierten Konfiguration zu halten.

Die US 2005/0216050 A1 offenbart eine Vorrichtung zum Entfernen einer Blutgefäßobstruktion, wobei die Vorrichtung ein Einführelement sowie ein mit dem Einführelement verbundenes, expandierbares Element aufweist.Aus der US 2006/0259005 A1 ist ein angioplastisches Kit bekannt, bei dem Schneidstrukturen beim Einführen in ein zu erweiterndes oder wiederzueröffnendes Körperhohlorgan mit dem Ballon eines Ballonkatheters oder mit dessen Enden verbunden sind.

Aufgabe der vorliegenden Erfindung ist es nun, ein medizinisches Kit bzw. Set zu schaffen, welches die einleitend beschriebenen Nachteile möglichst umgeht und insbesondere eine einfachere, sichere und kostengünstigere Behandlung von Patienten sowie eine einfachere Handhabung durch den behandelnden Mediziner erlaubt.

Diese Aufgabe wird erfindungsgemäß gelöst durch ein medizinisches Set bzw. Kit gemäß unabhängigem Anspruch 1. Bevorzugte Ausführungsformen sind in den abhängigen Ansprüchen 2 bis 13 definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein medizinisches Kit bzw. Set zur Anwendung bei der Behandlung eines krankhaft veränderten Hohlorgans. Das Kit bzw. Set kann somit auch als angioplastisches, insbesondere schneidangioplastisches (blade-angioplastisches), Kit bzw. Set bezeichnet werden.

Bevorzugt handelt es sich bei dem erfindungsgemäßen Kit bzw. Set um ein Kit bzw. Set zur Anwendung bei der Vorbereitung eines krankhaft veränderten Hohlorgans auf eine Rekanalisation, insbesondere Dilatation.

Unter dem Ausdruck "Rekanalisation" soll im Sinne der vorliegenden Erfindung eine artifizielle bzw. therapeutische Wiederöffnung eines obliterierten Hohlorgans, insbesondere Blutgefäßes, vorzugsweise einer Arterie, verstanden werden. Im Falle der Rekanalisation eines Blutgefäßes bewirkt die Rekanalisation zusätzlich eine Wiederdurchblutung des Blutgefäßes. Erfindungsgemäß handelt es sich bei der Rekanalisation vorzugsweise um eine Dilatation.

Weiterhin handelt es sich bei dem erfindungsgemäßen Kit bzw. Set vorzugsweise um ein Kit bzw. Set zur Anwendung bei der Rekanalisation, insbesondere Dilatation, eines krankhaft veränderten Hohlorgans.

Des Weiteren handelt es sich bei dem erfindungsgemäßen Kit bzw. Set bevorzugt um ein Kit bzw. Set zur Anwendung bei der Behandlung, insbesondere primären Behandlung, von Dissektionen einer Innenwandungsschicht eines krankhaft veränderten Hohlorgans.

Die krankhafte Veränderung des Hohlorgans beruht vorzugsweise auf einer Verengung (Stenose) oder Wiederverengung (Restenose) des Hohlorgans. Das Hohlorgan ist vorzugsweise ein Blutgefäß, insbesondere eine Arterie oder Vene.

Erfindungsgemäß besonders bevorzugt ist es, wenn es sich bei dem Hohlorgan um eine Arterie handelt. Die arterielle Wandung besteht grundsätzlich aus drei Schichten. Von der blutführenden Seite aus gesehen sind dies die sogenannte Tunica intima, Tunica media und die Tunica externa (auch als Tunica adventitia bezeichnet). Die Tunica intima stellt somit die Innenwandungsschicht, die Tunica media die Zwischenwandungsschicht und die Tunica externa bzw. Tunica adventitia die Außenwandungsschicht einer Arterie dar.

Das medizinische Kit bzw. Set weist ein Schneidelement und einen Einführkatheter auf.

Das Schneidelement ist für ein Einschneiden einer Innenwandungsschicht des Hohlorgans und/oder für ein Einschneiden von Ablagerungen, insbesondere arteriosklerotischen Plaque-Ablagerungen, welche sich auf einer Innenoberfläche des Hohlorgans befinden, gestaltet. Nach Einschneiden der Innenwandungsschicht kann dem Schneidelement zusätzlich eine wenigstens temporäre Stützfunktion zukommen. Die Stützfunktion bewirkt vorzugsweise eine wenigstens temporäre (vorübergehende) Stabilisierung bzw. Schienung eines aufgeweiteten (dilatierten) Hohlorgans. Die Stützfunktion beruht vorzugsweise darauf, dass das Schneidelement in der Lage ist, eine möglicherweise abgesprengte Innenwandungsschicht (Dissektion) des Hohlorgans an eine Zwischenwandungsschicht des Hohlorgans zu drücken bzw. zu pressen.

Der Einführkatheter ist für ein Einführen des Schneidelements in das Hohlorgan gestaltet. Zweckmäßigerweise weist der Einführkatheter hierzu einen Innendurchmesser auf, der ein wenigstens abschnittsweises Aufnehmen des Schneidelements gestattet.

In einer zweckmäßigen Ausführungsform gestattet der Innendurchmesser des Einführkatheters zusätzlich ein wenigstens abschnittsweises Aufnehmen eines Führungsdrahtes oder Führungskatheters.

Der Einführkatheter liegt vorzugsweise in Form eines flexiblen Kunststoffschlauches vor.

In einer unter Einführgesichtspunkten vorteilhaften Ausführungsform ist der Einführkatheter aus einem steiferen Material gebildet als das Schneidelement. Bevorzugt ist der Einführkatheter aus einem Kunststoffmaterial gebildet.

Das erfindungsgemäße Kit bzw. Set zeichnet sich dadurch aus, dass das Schneidelement in einem Einführzustand nicht mit einem Ballonkatheter verbunden ist.

Der Ausdruck "Einführzustand" bezeichnet im Sinne der vorliegenden Erfindung einen Zustand des Schneidelements, in welchem das Schneidelement dazu geeignet, insbesondere vorgesehen, ist, in das zu behandelnde Hohlorgan eingeführt zu werden. Bezüglich geeigneter Einführzustände des Schneidelements sei auf die nachfolgend beschriebenen Ausführungsformen zu dem Schneidelement verwiesen.

Die Erfindung beruht somit - im Gegensatz zum Stand der Technik - auf einer Separation von Schneidelement und Ballonkatheter. Ein Ballonkatheter stellt eine weitere Komponente des erfindungsgemäßen Kits bzw. Sets dar. Hierauf wird im Folgenden noch näher eingegangen werden.

Durch das erfindungsgemäße Kit bzw. Set lassen sich insbesondere die folgenden Vorteile realisieren:
> Das Schneidelement kann separat von dem Ballonkatheter in das zu behandelnde Hohlorgan eingeführt werden. Mit anderen Worten ist ein sequentielles Einführen von Schneidelement und dem Ballonkatheter in das Hohlorgan möglich. Dies vereinfacht den Einführvorgang und damit die Behandlung des Hohlorgans spürbar.
> Durch eine Separation von Schneidelement und Ballonkatheter ist es weiterhin möglich, mittels des Schneidelements ein gezieltes bzw. kontrolliertes Einschneiden der Innenwandungsschicht des Hohlorgans vorzunehmen. Denn nach Einführen und Aufblasen (Inflatieren) des Ballonkatheters wird das Einschneiden der Innenwandungsschicht des Hohlorgans mit besonderem Vorteil nur durch das Schneidelement verursacht, welches in der Regel eine kleinere Flächenausdehnung besitzt als der Ballonkatheter. Ein gezieltes bzw. kontrolliertes Einschneiden der Innenwandungsschicht des Hohlorgans wird vorzugsweise zusätzlich dadurch begünstigt, dass das Schneidelement nach Auswurf aus dem Einführkatheter lediglich abschnittsweise an der Innenoberfläche des Hohlorgans anliegt.
> Beim Inflatieren des in das Hohlorgan eingebrachten Ballonkatheters erfolgt eine gezielte Kraftbündelung auf das Schneidelement, wodurch das Einschneiden der Innenwandungsschicht des Hohlorgans sowie ein weitergehendes Inflatieren des Ballonkatheters erleichtert werden. Dadurch lässt sich mit besonderem Vorteil die Ausprägung von Dissektionen und Wandungshämatome reduzieren. Großflächige Trauma der Innenwandungsschicht des Hohlorgans können mithin vermieden werden. Ein reduziertes Hohlorganwandungstrauma bewirkt mit besonderem Vorteil eine verbesserte Offenheitsrate. Dies gilt insbesondere im Hinblick auf eine primäre Offenheitsrate, wodurch primäre Stentimplantationen entbehrlich werden können. Dadurch kann weiterhin auf die im Zusammenhang mit Stentimplantationen häufig notwendige Verabreichung von Thrombozytenaggregationshemmer verzichtet werden, wodurch Behandlungskosten eingespart werden können.
Entsprechendes gilt auch im Hinblick auf das Einschneiden von Ablagerungen, insbesondere arteriosklerotischen Plaque-Ablagerungen, welche sich auf der Innenoberfläche des Hohlorgans befinden. Insbesondere können die Ablagerungen mittels des Ballonkatheters besser dilatiert werden, d.h. stärker an oder in die Wandung des Hohlorgans gedrückt werden. Dies ist insbesondere im Hinblick auf eine Dilatation von härteren Plaque-Ablagerungen von Vorteil, wodurch eine gegebenenfalls doch erforderlich Stentimplantation erleichtert wird (Gefäßpräparation). Hinzu kommt, dass ein gezieltes bzw. kontrolliertes Einschneiden der Ablagerungen das Risiko senkt, dass gesundes Gewebe des Hohlorgans in Mitleidenschaft gezogen wird.
➢ Eine Behandlung, insbesondere primäre Behandlung, von Dissektionen wird ermöglicht.
➢ Durch ein gezieltes Einschneiden der Innenwandungsschicht des Hohlorgans können Eintrittskanäle für Medikamente erzeugt werden, wodurch selbst bei einer ablagerungsbedingt verdickten Innenwandungsschicht gewährleistet ist, dass die Medikamente an ihren Zielort gelangen. Im Falle von Arterien ist es in der Regel erforderlich, dass die Medikamente in die Tunica media diffundieren können, um eine Wirkung zu entfalten. Insgesamt lässt sich hierdurch die Wirksamkeit von Medikamenten, welche beispielsweise mittels eines medikamentenbeschichteten Ballonkatheters bereitgestellt werden können, erhöhen. Dies wiederum führt zu einer zusätzlichen Verbesserung der Offenheitsrate des Hohlorgans.
➢ Ein weiterer Vorteil besteht darin, dass das erfindungsgemäße Kit bzw. Set auch eine Behandlung eines Hohlorgans erlaubt, welches einen - physiologisch bedingt - unterschiedlichen Innendurchmesser besitzt. Denn das Schneidelement kann mit unterschiedlichen Ballonkathetern kombiniert werden. Mit besonderem Vorteil können Hohlorgane behandelt werden, welche Kalibersprünge von bis zu 3 mm aufweisen. Da zudem auf den Einsatz von (teuren) Schneid-Ballonkathetern verzichtet werden kann, können die Behandlungskosten spürbar reduziert werden.
➢ Die für marktübliche Schneid-Ballonkatheter erforderlichen Einführbestecke sind entbehrlich. Dadurch können große Zugänge am Patienten vermieden und ein Interventionsrisiko gesenkt werden.
➢ Auch das Entfernen des Schneidelements aus dem Hohlorgan vereinfacht sich, da das Schneidelement getrennt von dem Ballonkatheter entfernt werden kann. Das Risiko, dass das Schneidelement und der Ballonkatheter während des Entfernvorganges in unerwünschter Weise miteinander interagieren, beispielsweise dass es - wie eingangs erwähnt - zu einer Verdrillung zwischen Ballon und Schneidelement und Plaque kommt, besteht nicht.
   Weiterhin kann das Schneidelement als temporärer Schutz vor Dissektionen in dem Hohlorgan verbleiben, ohne dieses zu verschließen. Erfindungsgemäß ist beispielsweise ein Verbleib des Schneidelements in dem Hohlorgan während einigen Minuten vorstellbar.
➢ Insgesamt stellt die Erfindung somit ein leistungsstarkes, einfach zu handhabendes, patientenschonendes sowie kostensparendes Kit bzw. Set zur Anwendung auf dem Gebiet der Angioplastie zur Verfügung.

In einer bevorzugten Ausführungsform handelt es sich bei dem Schneidelement um ein wenigstens teilweise elastisches Schneidelement, d.h. um ein sich wenigstens teilweise elastisch rückstellend verhaltendes Schneidelement. Mit anderen Worten ist es erfindungsgemäß bevorzugt, wenn das Schneidelement bei Kraftbeaufschlagung wenigstens teilweise nachgibt und nach Wegfall der Kraftbeaufschlagung in eine ursprüngliche Form zurückkehrt.

Insbesondere kann es sich bei dem Schneidelement um ein vollständig elastisches Schneidelement, d.h. um ein sich vollständig elastisch rückstellend verhaltendes Schneidelement, handeln.

Das Schneidelement ist in einer zweckmäßigen Ausführungsform ein längliches Schneidelement.

Das Schneidelement weist in einer weiteren Ausführungsform einen Durchmesser von 0,003 mm bis 0,5 mm, insbesondere 0.1 mm bis 0.3 mm, auf. Die in diesem Absatz genannten, dünnen Durchmesser können mit besonderem Vorteil die Schneidfähigkeit des Schneidelements zusätzlich verbessern.

Gemäß einer weiteren Ausführungsform weist das Schneidelement in einem kraftbeaufschlagten Zustand eine gestreckte, insbesondere ein teilweise gestreckte bzw. teilgestreckte, Form auf. Anders ausgedrückt, ist das Schneidelement gemäß einer weiteren Ausführungsform durch Kraftbeaufschlagung in eine gestreckte, insbesondere teilweise gestreckte bzw. teilgestreckte, Form überführbar. Dies erleichtert mit besonderem Vorteil ein Aufnehmen des Schneidelements in den Einführkatheter und damit ein Einführen des Schneidelements in das zu behandelnde Hohlorgan.

Das Schneidelement weist in einer weitergehenden Ausführungsform in einem krafbeaufschlagten Zustand eine in Längsrichtung durchgehend gestreckte, insbesondere in Längsrichtung durchgehend teilweise gestreckte bzw. teilgestreckte, Form auf.

Die beiden vorherigen Ausführungsformen beschreiben bevorzugte Einführzustände für das Schneidelement.

In einem kraftbeaufschlagten, insbesondere gestreckten, Zustand kann das Schneidelement eine Länge von 50 mm bis 200 mm, insbesondere 100 mm bis 200 mm, aufweisen.

In einer weiteren Ausführungsform weist das Schneidelement in einem nicht kraftbeaufschlagten Zustand eine gegenüber einem kraftbeaufschlagten Zustand expandierte und insbesondere längenverkürzte Form auf.

In einem nicht kraftbeaufschlagten Zustand kann das Schneidelement eine Länge von 40 mm bis 180 mm, insbesondere 80 mm bis 180 mm, aufweisen.

Das Schneidelement ist in einer weiteren Ausführungsform in einem nicht kraftbeaufschlagten Zustand spiralförmig ausgestaltet, d.h. weist eine spiralförmige Struktur auf oder liegt in Form einer Spirale vor.

Gemäß einer besonders bevorzugten Ausführungsform ist das Schneidelement in einem nicht kraftbeaufschlagten Zustand helikal bzw. wendelförmig ausgestaltet, d.h. weist eine helikale bzw. wendelförmige Struktur auf oder liegt in Form einer Helix bzw. Wendel vor.

In einer weitergehenden Ausführungsform weist die Helix bzw. Wendel eine Ganghöhe auf, welche einem Innendurchmesser des behandelnden Hohlorgans entspricht. Beispielsweise kann die Helix bzw. Wendel eine Ganghöhe von 2 mm bis 20 mm, insbesondere 3 mm bis 8 mm, aufweisen.

Bevorzugt ist das Schneidelement zugfederförmig, insbesondere schraubenzugfederförmig, ausgestaltet, d.h. weist eine zugfederförmige, insbesondere schraubenzugfederförmige, Struktur auf oder liegt in Form einer Zugfeder, insbesondere Schraubenzugfeder, vor.

Das Schneidelement ist unter Handhabungsgesichtspunkten wenigstens abschnittsweise, vorzugsweise vollständig, in dem Einführkatheter enthalten. Erfindungsgemäß kann es dabei insbesondere vorgesehen sein, dass lediglich das Schneidelement in dem Einführkatheter enthalten ist.

Bevorzugt liegt das Schneidelement innerhalb des Einführkatheters in einem kraftbeaufschlagten Zustand vor. Bei der das Schneidelement beaufschlagenden Kraft handelt es sich vorzugsweise um eine von einer Wandung des Einführkatheters erzeugte Gegenkraft zu einer Kraft, welche das Schneidelement seinerseits auf die Wandung des Einführkatheters ausübt.

Das Schneidelement liegt innerhalb des Einführkatheters in einem gestreckten, insbesondere teilweise gestreckten bzw. teilgestreckten, Zustand vor.

Außerhalb des Einführkatheters, insbesondere nach einem Auswerfen aus dem Einführkatheter, liegt das Schneidelement dagegen vorzugsweise in Form einer Spirale oder, was erfindungsgemäß besonders bevorzugt ist, in Form einer Helix bzw. Wendel vor. Eine helix- bzw. wendelförmige Ausgestaltung des Schneidelements hat gegenüber einer spiralförmigen Ausgestaltung den Vorteil, dass sich das Schneidelement nach Auswurf aus dem Einführkatheter gleichmäßiger an die Innenoberfläche des Hohlorgans anlegen kann.

Das Schneidelement kann in einer weiteren Ausführungsform einen runden, d.h. kreisförmigen, Querschnitt besitzen.

In einer unter Schneidgesichtspunkten vorteilhaften Ausführungsform besitzt das Schneidelement einen unrunden, d.h. nicht kreisförmigen, Querschnitt. Das Schneidelement kann dabei insbesondere einen polygonalen Querschnitt besitzen. Beispielsweise kann das Schneidelement einen dreieckförmigen, viereckförmigen (quadratischen oder rechteckförmigen), fünfeckförmigen, sechseckförmigen oder sternenförmigen Querschnitt besitzen.

In einer unter Schneidgesichtspunkten besonders vorteilhaften Ausführungsform kann das Schneidelement einen Querschnitt aufweisen, welcher auf einer Seite atraumatisch, insbesondere flach, und auf einer gegenüberliegenden Seite traumatisch, insbesondere spitz oder zugespitzt, ausgestaltet ist. Ein entsprechend geeigneter Querschnitt ist beispielsweise ein dreieckförmiger Querschnitt. Alternativ kann es sich bei dem Querschnitt des Schneidelements um einen T- oder L-förmigen Querschnitt handeln. Mit anderen Worten kann es erfindungsgemäß vorgesehen sein, dass es sich bei dem Schneidelement um ein Schneidelement mit einem T- oder L-Profil handelt.

Weist das Schneidelement in einem nicht kraftbeaufschlagten Zustand eine spiralförmige oder helikale Struktur auf oder liegt das Schneidelement in einem nicht kraftbeaufschlagten Zustand in Form einer Spirale oder Helix vor, so ist es bevorzugt, wenn die im vorherigen Absatz erwähnte traumatische Querschnittsseite nicht in das Innere der spiralförmigen oder helikalen Struktur/der Spirale oder Helix, sondern in die entgegengesetzte Richtung gerichtet ist.

In einer weiteren unter Schneidgesichtspunkten vorteilhaften Ausführungsform ist das Schneidelement um die eigene Längsachse verdreht. Aufgrund der Verdrehung erhält die Oberfläche des Schneidelements eine Profilierung bzw. Strukturierung, wodurch das Schneidvermögen des Schneidelements zusätzlich verbessert werden kann.

In einer bevorzugten Ausführungsform weist das Schneidelement wenigstens einen Draht auf oder besteht aus wenigstens einem Draht.

Erfindungsgemäß besonders bevorzugt ist es, wenn das Schneidelement drahtförmig ausgestaltet ist, d.h. wenn das Schneidelement eine drahtförmige Struktur aufweist, oder es sich bei dem Schneidelement um einen Draht handelt bzw. das Schneidelement in Form eines Drahts vorliegt. In dieser Ausführungsform kann das Schneidelement auch als Schneiddraht bezeichnet werden.

Gemäß einer weiteren Ausführungsform kann das Schneidelement wenigstens eine Faser aufweisen oder aus wenigstens einer Faser bestehen. Das Schneidelement kann insbesondere faserförmig ausgestaltet sein, d.h. eine faserförmige Struktur aufweisen oder in Form einer Faser vorliegen.

Gemäß einer weiteren Ausführungsform weist das Schneidelement wenigstens einen Faden auf oder besteht aus wenigstens einem Faden. Erfindungsgemäß kann es insbesondere vorgesehen sein, dass das Schneidelement fadenförmig ausgestaltet ist, d.h. eine fadenförmige Struktur aufweist oder in Form eines Fadens vorliegt.

In einer weiteren Ausführungsform weist das Schneidelement mehrere Drähte, Fasern oder Fäden auf oder besteht aus mehreren Drähten, Fasern und/oder Fäden, welche vorzugsweise in Längsrichtung des Schneidelements verdrillt sind. Die Verdrillung der Drähte, Fasern und/oder Fäden bewirkt eine Strukturierung bzw. Profilierung der Oberfläche des Schneidelements, wodurch ebenfalls eine Verbesserung des Schneidvermögens des Schneidelements herbeigeführt werden kann.

In einer unter Einführgesichtspunkten vorteilhaften Ausführungsform weist das Schneidelement an seinem distalen Ende ein Verbindungseinrichtung auf, welche für ein Verbinden, insbesondere temporäres (vorübergehendes) Verbinden, mit einem Führungsdraht oder Führungskatheter gestaltet ist. Die Verbindungseinrichtung kann beispielsweise haken-, schlaufen-, hülsen- oder spiralfömig ausgebildet sein.

Erfindungsgemäß kann es insbesondere vorgesehen sein, dass das Schneidelement im Bereich seines distalen Endes eine Form annimmt, welche ein Verbinden, insbesondere temporäres Verbinden, mit einem Führungsdraht oder Führungskatheter gestattet. Beispielsweise kann das Schneidelement an seinem distalen Ende haken-, schlaufen-, hülsen- oder spiralfömig ausgebildet sein.

In einer weiteren Ausführungsform weist das Schneidelement ein Formgedächtnismaterial auf oder besteht aus einem solchen Material. Bei dem Formgedächtnismaterial kann es sich um ein Formgedächtnismetall oder eine Formgedächtnislegierung handeln. Bezüglich geeigneter Formgedächtnismaterialien wird auf die im Folgenden genannten Materialien Bezug genommen.

In einer weiteren Ausführungsform weist das Schneidelement ein Metall auf oder besteht aus einem Metall. Das Metall ist vorzugsweise ausgewählt aus der Gruppe umfassend Eisen, Gold, Nickel, Niob, Platin, Silber, Tantal, Titan, Vanadium und Zirkonium.

In einer weiteren Ausführungsform weist das Schneidelement eine Legierung auf oder besteht aus einer Legierung. Die Legierung ist vorzugsweise ausgewählt aus der Gruppe umfassend rostfreie Stähle wie Chrom-Nickel-Stähle, Kobalt-Basis-Legierungen wie Kobalt-Chrom-Legierungen oder Kobalt-Chrom-Molybdän-Legierungen und Titanlegierungen wie Nitinol.

Eine unter medizinischen Gesichtspunkten vorteilhafte Kobalt-Chrom-Molybdän-Legierung ist beispielsweise unter der Bezeichnung Vitallium bekannt. Diese Metalllegierung besitzt einen Kobaltgehalt von ca. 60 Gew.-%, einen Chromgehalt von 25-30 Gew.-% sowie einen Molybdängehalt von 5-10 Gew.-%, jeweils bezogen auf das Gesamtgewicht der Legierung. Gegebenenfalls kann die Legierung andere Elemente in geringen Anteilen besitzen.

Gemäß einer besonders bevorzugten Ausführungsform weist das Schneidelement eine Nickel-Titan-Legierung auf oder besteht aus einer solchen Legierung.

Besonders bevorzugt weist das Schneidelement Nitinol auf oder besteht aus Nitinol. Nitinol zählt zu den Formgedächtnislegierungen und stellt eine korrosionsbeständige, hochfeste Nickel-Titan-Legierung, in der Regel mit einem Nickelgehalt von ca. 55%, dar.

In einer besonders bevorzugten Ausführungsform handelt es sich bei dem Schneidelement um einen Nitinoldraht, vorzugsweise um einen elastischen bzw. sich elastisch rückstellend verhaltenden Nitinoldraht.

In einer weiteren Ausführungsform weist das Schneidelement ein Polymer oder Polymerblend auf. Insbesondere kann das Schneidelement aus einem Polymer oder Polymerblend bestehen.

In einer unter Behandlungsgesichtspunkten vorteilhaften Ausführungsform ist das Schneidelement wenigstens teilweise mit einem Additiv versehen, insbesondere beschichtet. Bei dem Additiv kann es sich um ein röntgensichtbares Additiv, wie beispielsweise Bariumsulfat, handeln. Auf diese Weise ist eine Positionierung des Schneidelements in das zu behandelnde Hohlorgan unter Röntgenkontrolle möglich.

Zusätzlich oder alternativ kann das Schneidelement mit einem Medikament, insbesondere einem Zytostatikum, vorzugsweise Paclitaxel, versehen, insbesondere beschichtet, sein. Die Additivierung des Schneidelements mit einem Zytostatikum hat den Vorteil, dass sich hierdurch das Risiko einer Restenose zusätzlich verringern lässt.

In einer weiteren Ausführungsform weist das medizinische Kit bzw. Set ferner einen Führungsdraht auf. Der Führungsdraht ist vorzugsweise dazu vorgesehen, das Einführen des Schneidelements in das zu behandelnde Hohlorgan zu ermöglichen oder zu erleichtern. Um die Gleiteigenschaften des Führungsdrahts zu verbessern, kann es erfindungsgemäß weiterhin vorgesehen sein, dass der Führungsdraht mit einer hydrophilen Beschichtung versehen ist.

In einer weiteren Ausführungsform weist das medizinische Kit bzw. Set ferner einen Führungskatheter auf. Der Führungskatheter ist vorzugsweise dazu vorgesehen, das Einführen des Schneidelements in das zu behandelnde Hohlorgan zu ermöglichen oder zu erleichtern. Um die Gleiteigenschaften des Führungskatheters zu verbessern, kann es erfindungsgemäß weiterhin vorgesehen sein, dass der Führungskatheter mit einer hydrophilen Beschichtung versehen ist.

Das medizinische Kit bzw. Set weist ferner wenigstens einen Ballonkatheter, insbesondere wenigstens einen medikamentenbeschichteten Ballonkatheter, auf.

In einer weiteren Ausführungsform weist das medizinische Kit bzw. Set ferner mehrere Ballon-Katheter auf. Die Ballonkatheter können sich dabei hinsichtlich ihres Ballondurchmessers im inflatierten Zustand unterscheiden.

Erfindungsgemäß kann es insbesondere vorgesehen sein, dass das medizinische Kit bzw. Set ferner wenigstens einen medikamentenbeschichteten Ballonkatheter sowie wenigstens einen unbeschichteten Ballonkatheter aufweist.

In einer weiteren Ausführungsform kann das medizinische Kit bzw. Set ferner ein Einführbesteck, eine Inflationsspritze, eine Rotations- und Greifhilfe für Führungsdrähte (Torquer) und/oder Anschlussstücke aufweisen.

Ferner wird ein exemplarisches Verfahren zur Behandlung eines krankhaft veränderten Hohlorgans offenbart aufweisend die folgenden Schritte:
a) Einführen eines Schneidelements mittels eines Einführkatheters in das Hohlorgan, wobei das Schneidelement in einem Einführzustand nicht mit einer Ballonaußenfläche eines Ballonkatheters verbunden ist, und
b) Entfernen des Einführkatheters aus dem Hohlorgan unter gleichzeitigem Auswerfen des Schneidelements in ein Lumen des Hohlorgans.

Das Verfahren kann auch als angioplastisches, insbesondere schneidangioplastisches (blade-angioplastisches), Verfahren bezeichnet werden.

Das Verfahren ist vorzugsweise zur Anwendung bei der Vorbereitung eines krankhaft veränderten Hohlorgans auf eine Rekanalisation, insbesondere Dilatation, vorgesehen.

Weiterhin ist es bevorzugt, wenn das Verfahren zur Anwendung bei der Rekanalisation, insbesondere Dilatation, eines krankhaft veränderten Hohlorgans vorgesehen ist.

Des Weiteren ist es bevorzugt, wenn das Verfahren zur Behandlung, insbesondere primären Behandlung, von Dissektionen einer Innenwandungsschicht des Hohlorgans vorgesehen ist.

Das Einführen des Schneidelements wird in einer bevorzugten Form mittels eines Führungsdrahtes und/oder -katheters vorgenommen. Der Führungsdraht und/oder Führungskatheter werden vorzugsweise vor dem Einführen des Schneidelements in das Hohlorgan, d.h. vor Durchführen des Schrittes a), in das Hohlorgan eingeführt.

Bevorzugt nimmt das Schneidelement beim Auswerfen in das Lumen des Hohlorgans eine Form an, welche sich abschnittsweise, insbesondere nur abschnittsweise, an eine Innenoberfläche des Hohlorgans anlegt. Bei der Form handelt es sich vorzugsweise um eine helicale bzw. wendelförmige Form.

In einer besonders bevorzugten Form wird nach dem Entfernen des Einführkatheters aus dem Hohlorgan, d.h. nach Durchführen von Schritt b), ein Ballonkatheter in das Hohlorgan eingeführt und anschließend inflatiert. Durch das Inflatieren des Ballonkatheters wird das Schneidelement an eine Innenoberfläche des Hohlorgans gedrückt bzw. gepresst, wodurch eine Innenwandungsschicht des Hohlorgans eingeschnitten und/oder auf einer Innenoberfläche des Hohlorgans vorhandene Ablagerungen, insbesondere arteriosklerotische Plaque-Ablagerungen, eingeschnitten und an oder in eine Wandung des Hohlorgans gepresst werden. Bei dem Ballonkatheter kann es sich insbesondere um einen medikamentenbeschichteten Ballonkatheter handeln. Beispielsweise kann der Ballonkatheter mit einem Medikament, insbesondere Zytostatikum, wie Paclitaxel, beschichtet sein. Im Falle des Einschneidens der Innenwandungsschicht des Hohlorgans kann das Medikament aufgrund der hierbei erzeugten Kanäle besser in eine Zwischenwandungsschicht des Hohlorgans gelangen und dort seine Wirkung entfalten. Das Inflatieren des Ballonkatheters kann beispielsweise mit Druckgas oder Flüssigkeit erfolgen.

Nach Inflatieren des Ballonkatheters wird dieser deflatiert und aus dem Hohlorgan entfernt. Die Behandlung kann entweder mit demselben Ballonkatheter oder einem anderen Ballonkatheter, beispielsweise einem Ballonkatheter, dessen Ballon im inflatierten Zustand einen anderen Durchmesser besitzt, oder einem medikamentenbeschichteten Ballonkatheter, wiederholt oder fortgesetzt werden.

In einer weiteren exemplarischen Ausführungsform wird das Schneidorgan nach Entfernen des Ballonkatheters für eine temporäre Schienung bzw. Stützung in dem Hohlorgan belassen.

In einer alternativen Form wird das Schneidelement unmittelbar nach Entfernen des Ballonkatheters aus dem Hohlorgan entfernt.

Bezüglich weiterer Merkmale und Vorteile des Schneidelements des Einführkatheters, eines ggf. zusätzlich vorgesehenen Führungsdrahtes, eines ggf. zusätzlich vorgesehenen Führungskatheters sowie eines oder mehrerer ggf. zusätzlich vorgesehener Ballonkatheter, wird zur Vermeidung von unnötigen Wiederholungen vollständig auf die im Rahmen des ersten Erfindungsaspektes gemachten Ausführungen Bezug genommen.

Weitere Vorteile und Merkmale der Erfindung ergeben sich aus der nachfolgenden Beschreibung bevorzugter Ausführungsbeispiele der Erfindung, die anhand der Figuren dargestellt sind, sowie aus den Ansprüchen.

In den Figuren ist Folgendes schematisch gezeigt:
- Fig. 1:: eine Ausführungsform eines erfindungsgemäßen Kits bzw. Sets,
- Fig. 2 - 4:: eine Behandlung eines krankhaft veränderten Hohlorgans mittels einer Ausführungsform eines erfindungsgemäßen Kits bzw. Sets.

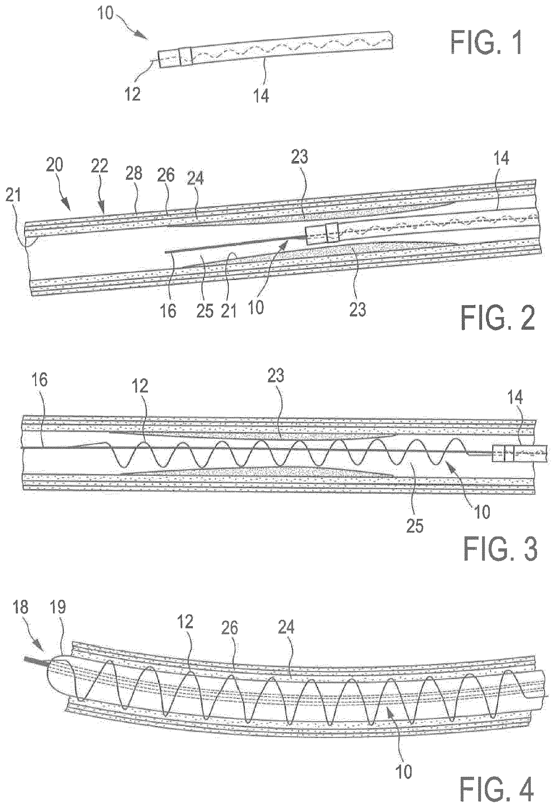

Fig. 1 zeigt schematisch ein erfindungsgemäßes Kit bzw. Set 10 zur Behandlung eines krankhaft veränderten Hohlorgans. Das Kit bzw. Set 10 weist ein drahtförmiges Schneidelement 12 sowie einen Einführkatheter 14 auf.

Das Schneidelement 12 ist für ein gezieltes bzw. kontrolliertes Einschneiden einer Innenwandungsschicht eines Hohlorgans und/oder für ein Einschneiden von auf einer Innenoberfläche eines Hohlorgans befindlichen Ablagerungen, insbesondere arteriosklerotischen Plaque-Ablagerungen, vorgesehen.

Der Einführkatheter 14 ist zum Einführen des Schneidelements 12 in das zu behandelnde Hohlorgan vorgesehen.

Bei dem Schneidelement 12 handelt es sich bevorzugt um einen elastischen bzw. sich elastisch rückstellend verhaltenden Draht, insbesondere aus Nitinol. Das Schneidelement 12 liegt in einem kraftbeaufschlagten Zustand vorzugsweise in einer gestreckten Form und in einem nicht kraftbeaufschlagten Zustand vorzugsweise in Form einer Helix bzw. Wendel vor. Mit anderen Worten ist das Schneidelement 12 bevorzugt schraubenzugfederförmig ausgestaltet.

Das Schneidelement 12 ist unter Handhabungsgesichtspunkten bereits in dem Einführkatheter 14 enthalten. Das Schneidelement 12 liegt innerhalb des Einführkatheters 14 in einer gestreckten Form vor. Bei der gestreckten Form muss es sich nicht um eine vollständig, d.h. linear, gestreckte Form des Schneidelements 12 handeln. Erfindungsgemäß kann es sogar bevorzugt sein, wenn das Schneidelement 12 innerhalb des Einführkatheters 14 nur in einer teilweise verstreckten bzw. teilverstreckten Form vorliegt. Dies begünstigt nämlich die Rückkehr des Schneidelements 12 in eine ursprüngliche Form, bevorzugt in eine Helix- oder Wendelform, des Schneidelements 12.

Nachfolgend wird anhand der Fig. 2 bis 4 beispielhaft die Behandlung eines krankhaft veränderten Hohlorgans 20 mittels eines erfindungsgemäßen Kits bzw. Sets 10 gemäß Fig. 1 beschrieben.

Um die in den Fig. 2 bis 4 dargestellte Behandlung durchführen zu können, ist es von Vorteil, wenn das erfindungsgemäße Kit bzw. Set 10 ferner einen Führungsdraht 16 sowie einen Ballonkatheter 18 aufweist.

Das zu behandelnde Hohlorgan 20 besitzt eine Wandung 22, welche sich aus einer Innenwandungsschicht 24, einer Zwischenwandungsschicht 26 sowie einer Außenwandungsschicht 28 zusammensetzt. Handelt es sich bei dem Hohlorgan 20 um eine Arterie, stellt die Innenwandungsschicht 24 die sogenannte Tunica intima, die Zwischenwandungsschicht 26 die sogenannte Tunica media und die Außenwandungsschicht 28 die sogenannte Tunica externa dar.

Die krankhafte Veränderung des Hohlorgans 20 besteht vorzugsweise in einer Stenose (oder Restenose), welche durch Ablagerungen 23 (oder gebildetes Narbengewebe 23 im Falle einer Restenose) auf der Innenoberfläche 21 des Hohlorgans 20 entstehen kann.

Zunächst wird der Einführkatheter 14 mit dem darin enthaltenen, in (teil-)gestreckter Form vorliegenden, Schneidelement 12 mittels des Führungsdrahtes 16 in das Lumen 25 des zu behandelnden Hohlorgans 20 eingeführt (siehe Figur 2).

Sobald die Stenose (oder Restenose) erreicht ist, wird der Einführkatheter 14 zurückgezogen und das Schneidelement 12 in das Hohlorgan 20 ausgeworfen. Beim Auswerfen aus dem Einführkatheter 14 nimmt das Schneidelement 12, vorzugsweise einer elastischen Rückstellkraft folgend, eine helikale bzw. wendelförmige Form an (siehe Figur 3). Alternativ kann das Schneidelement 12 beim Auswerfen aus dem Einführkatheter 14 aufgrund eines Formgedächtnisses eine helikale bzw. wendelförmige Form annehmen. In dieser Form liegt das Schneidelement 12 vorzugsweise abschnittsweise an der Innenoberfläche 21 des Hohlorgans 20 an.

Durch ein nachfolgendes Einführen des Ballonkatheters 18 und Aufblasen seines Ballons 19 wird die Innenwandungsschicht 24 durch das Schneidelement 12 eingeschnitten (siehe Figur 4). Das Einschneiden der Innenwandungsschicht 24 erfolgt dabei nur an den Stellen, an welchen das Schneidelement 12 die Innenoberfläche 21 des Hohlorgans 20 vor dem Aufblasen des Ballons 19 berührt hat. Dadurch ist ein kontrolliertes Einschneiden der Innenwandungsschicht 24 möglich. Auf diese Weise ist eine Behandlung der Stenose (oder Reststenose) ohne Verursachung eines großflächigen Wandungstraumas möglich.

Anschließend kann der Ballon 19 deflatiert und der Ballonkatheter 18 wieder aus dem Hohlorgan 20 entfernt, insbesondere zurückgezogen, werden.

Danach kann das Schneidelement 12 ebenfalls aus dem Hohlorgan 20 entfernt, insbesondere zurückgezogen, werden. Gegebenenfalls kann das Schneidelement 12 vor seinem Entfernen für eine temporäre Schienung in dem Hohlorgan 20 belassen werden.

Die Behandlung kann bei Bedarf mit dem Ballonkatheter 18 (ohne erneutes Einführen des Schneidelements 12 in das Hohlorgan 20) wiederholt oder mit einem anderen Ballonkatheter, beispielsweise einem medikamentenbeschichteten Ballonkatheter, fortgesetzt werden. Die Verwendung eines medikamentenbeschichteten Ballonkatheters hat den Vorteil, dass die Medikamente aufgrund der durch das Schneidelement 12 in die Innenwandungsschicht 24 erzeugten Einschnitte leichter in die Zwischenwandungsschicht 26 des Hohlorgans 20 gelangen und dort ihre Wirkung entfalten können.

## Patentansprüche

1. Medizinisches Kit bzw. Set (10) zur Anwendung bei der Behandlung eines krankhaft veränderten Hohlorgans (20), aufweisend ein Schneidelement (12), welches für ein Einschneiden einer Innenwandungsschicht (24) des Hohlorgans (20) und/oder für ein Einschneiden von Ablagerungen (23) auf einer Innenoberfläche (21) des Hohlorgans gestaltet ist, und einen Einführkatheter (14), welcher für ein Einführen des Schneidelements (12) in das Hohlorgan (20) gestaltet ist, wobei das Schneidelement (12) in dem Einführkathether (14) enthalten ist, wobei das Schneidelement (12) innerhalb des Einführkatheters (14) in einem gestreckten Zustand vorliegt, **dadurch gekennzeichnet, dass** das Schneidelement (12) in einem Einführzustand nicht mit einem Ballonkatheter (18) verbunden ist, wobei
das Kit bzw. Set (10) ferner wenigstens einen Ballonkatheter (18) aufweist, wobei der Ballon (19) des Ballonkatheters (18) und das Schneidelement (12) angepasst sind, um ein Einführen des Ballons (19) in das Schneidelement (12) zu ermöglichen, wenn sich das Schneidelement (12) im eingesetzten und einem expandierten Zustand befindet, so dass durch Aufblasen des Ballons (19) die Innenwandungsschicht (24) und/oder die Ablagerungen (23) durch das Schneidelement (12) eingeschnitten werden/wird, wobei die Ablagerungen (23) an oder in die Wandung (22) des Hohlorgans (20) gepresst werden.

2. Medizinisches Kit bzw. Set (10) nach Anspruch 1, **dadurch gekennzeichnet, dass** das Schneidelement (12) wenigstens teilweise ein sich elastisch rückstellend verhaltendes Schneidelement (12) ist.

3. Medizinisches Kit bzw. Set (10) nach Anspruch 1 oder 2, **dadurch gekennzeichnet, dass** das Schneidelement (12) in einem kraftbeaufschlagten Zustand eine gestreckte Form aufweist.

4. Medizinisches Kit bzw. Set (10) nach einem der vorhergehenden Ansprüche, **dadurch gekennzeichnet, dass** das Schneidelement (12) in einem nicht kraftbeaufschlagten Zustand eine gegenüber einem kraftbeaufschlagten Zustand expandierte und insbesondere längenverkürzte Form aufweist.

5. Medizinisches Kit bzw. Set (10) nach einem der vorhergehenden Ansprüche, **dadurch gekennzeichnet, dass** das Schneidelement (12) in einem nicht kraftbeaufschlagten Zustand helikal bzw. wendelförmig ausgestaltet ist.

6. Medizinisches Kit bzw. Set (10) nach einem der vorhergehenden Ansprüche, **dadurch gekennzeichnet, dass** das Schneidelement (12) zugfederförmig, insbesondere schraubenzugfederförmig, ausgestaltet ist.

7. Medizinisches Kit bzw. Set (10) nach einem der vorhergehenden Ansprüche, **dadurch gekennzeichnet, dass** das Schneidelement (12) ein Metall aufweist oder aus einem Metall besteht, welches vorzugsweise ausgewählt ist aus der Gruppe umfassend Eisen, Gold, Nickel, Niob, Platin, Silber, Tantal, Titan, Vanadium und Zirkonium.

8. Medizinisches Kit bzw. Set (10) nach einem der Ansprüche 1 bis 6, **dadurch gekennzeichnet, dass** Schneidelement (12) eine Legierung aufweist oder aus einer Legierung besteht, welche vorzugsweise ausgewählt ist aus der Gruppe umfassend rostfreie Stähle wie Chrom-Nickelstähle, Cobalt-Basis-Legierungen wie Cobalt-Chrom-Molybdän-Legierungen (Vitallium) und Titanlegierungen wie Nitinol.

9. Medizinisches Kit bzw. Set (10) nach einem der vorhergehenden Ansprüche, **dadurch gekennzeichnet, dass** Schneidelement (12) drahtförmig ausgestaltet ist, vorzugsweise ein Nitinoldraht ist.

10. Medizinisches Kit bzw. Set (10) nach einem der vorhergehenden Ansprüche, **dadurch gekennzeichnet, dass** das Schneidelement (12) wenigstens teilweise mit einem Additiv, insbesondere einem röntgensichtbaren Additiv oder einem Medikament, beschichtet ist.

11. Medizinisches Kit bzw. Set (10) nach einem der vorhergehenden Ansprüche, **dadurch gekennzeichnet, dass** das Kit bzw. Set (10) ferner einen Führungsdraht (16), insbesondere einen mit einer hydrophilen Beschichtung versehenen Führungsdraht (16), aufweist.

12. Medizinisches Kit bzw. Set (10) nach einem der vorhergehenden Ansprüche, **dadurch gekennzeichnet, dass** das Kit bzw. Set (10) ferner einen Führungskatheter, insbesondere einen mit einer hydrophilen Beschichtung versehenen Führungskatheter, aufweist.

13. Medizinisches Kit bzw. Set (10) nach einem der vorhergehenden Ansprüche, **dadurch gekennzeichnet, dass** der wenigstens eine Ballonkatheter (18) wenigstens ein medikamentenbeschichteter Ballonkatheter (18) ist.

## Claims

1. Medical kit or set (10) for use in the treatment of a diseased hollow organ (20), having a cutting element (12), which is designed to cut into an inner wall layer (24) of the hollow organ (20) and/or to cut into deposits (23) on an inner surface (21) of the hollow organ, and an insertion catheter (14), which is designed for inserting the cutting element (12) into the hollow organ (20), the cutting element (12) being contained in the insertion catheter (14), the cutting element (12) being in an extended state inside the insertion catheter (14), **characterized in that** the cutting element (12), in an insertion state, is not connected to a balloon catheter (18), wherein the kit or set (10) moreover has at least one balloon catheter (18), wherein the balloon (19) of the balloon catheter (18) and the cutting element (12) are adapted to permit insertion of the balloon (19) into the cutting element (12) when the cutting element (12) is located in the inserted and an expanded state, such that, through inflation of the balloon (19), the cutting element (12) cuts into the inner wall layer (24) and/or the deposits (23), wherein the deposits (23) are pressed onto or into the wall (22) of the hollow organ (20).

2. Medical kit or set (10) according to Claim 1, **characterized in that** the cutting element (12) is at least in part an elastically restoring cutting element (12).

3. Medical kit or set (10) according to Claim 1 or 2, **characterized in that** the cutting element (12), in a state when subjected to force, has an elongate shape.

4. Medical kit or set (10) according to one of the preceding claims, **characterized in that** the cutting element (12), in a state when not subjected to force, has an expanded and in particular length-reduced shape as compared to a state when subjected to force.

5. Medical kit or set (10) according to one of the preceding claims, **characterized in that** the cutting element (12), in a state when not subjected to force, has a helical shape.

6. Medical kit or set (10) according to one of the preceding claims, **characterized in that** the cutting element (12) has the shape of a tensile spring, in particular a helical tensile spring.

7. Medical kit or set (10) according to one of the preceding claims, **characterized in that** the cutting element (12) comprises a metal or consists of a metal which is preferably chosen from the group comprising iron, gold, nickel, niobium, platinum, silver, tantalum, titanium, vanadium and zirconium.

8. Medical kit or set (10) according to one of Claims 1 to 6, **characterized in that** the cutting element (12) comprises an alloy or consists of an alloy which is preferably chosen from the group comprising stainless steels such as chromium-nickel steels, cobalt-based alloys such as cobalt-chromium-molybdenum alloys (Vitallium) and titanium alloys such as nitinol.

9. Medical kit or set (10) according to one of the preceding claims, **characterized in that** the cutting element (12) is in the shape of a wire, preferably a nitinol wire.

10. Medical kit or set (10) according to one of the preceding claims, **characterized in that** the cutting element (12) is at least partially coated with an additive, in particular with a radiopaque additive or a medicament.

11. Medical kit or set (10) according to one of the preceding claims, **characterized in that** the kit or set (10) further comprises a guide wire (16), in particular a guide wire (16) provided with a hydrophilic coating.

12. Medical kit or set (10) according to one of the preceding claims, **characterized in that** the kit or set (10) further comprises a guide catheter, in particular a guide catheter provided with a hydrophilic coating.

13. Medical kit or set (10) according to one of the preceding claims, **characterized in that** the at least one balloon catheter (18) is at least one balloon catheter (18) coated with medicament.

## Revendications

1. Trousse médicale ou ensemble médical (10) destiné à être utilisé dans le traitement d'un organe creux (20) pathologiquement altéré, ladite trousse médicale ou ledit ensemble médical comportant un élément coupant (12) qui est conçu pour inciser une couche de paroi intérieure (24) de l'organe creux (20) et/ou inciser des dépôts (23) sur une surface intérieure (21) de l'organe creux, et un cathéter d'insertion (14) qui est conçu pour insérer l'élément coupant (12) dans l'organe creux (20), l'élément coupant (12) étant contenu dans le cathéter d'insertion (14), l'élément coupant (12) étant dans un état étiré à l'intérieur du cathéter d'insertion (14), caractérisé(e) en ce que l'élément coupant (12) n'est pas relié à un cathéter à ballonnet (18) dans un état d'insertion, la trousse ou l'ensemble (10) comportant en outre au moins un cathéter à ballonnet (18), le ballonnet (19) du cathéter à ballonnet (18) et l'élément coupant (12) étant adaptés pour permettre l'insertion du ballonnet (19) dans l'élément coupant (12) lorsque l'élément coupant (12) se trouve dans l'état inséré et un état expansé de sorte que la couche de paroi intérieure (24) et/ou les dépôts (23) soient incisés par l'élément coupant (12) par gonflage du ballonnet (19), les dépôts (23) étant pressés contre ou dans la paroi (22) de l'organe creux (20).

2. Trousse médicale ou ensemble médical (10) selon la revendication 1, caractérisé(e) en ce que l'élément coupant (12) est au moins partiellement un élément coupant (12) qui se comporte de manière élastiquement rétractable.

3. Trousse médicale ou ensemble médical (10) selon la revendication 1 ou 2, caractérisé(e) en ce que l'élément coupant (12) a une forme étirée dans un état contraint.

4. Trousse médicale ou ensemble médical (10) selon l'une des revendications précédentes, caractérisé(e) en ce que l'élément coupant (12) a dans un état non contraint une forme expansée et notamment raccourcie en longueur par rapport à un état contraint.

5. Trousse médicale ou ensemble médical (10) selon l'une des revendications précédentes, caractérisé(e) en ce que l'élément coupant (12) est conçu en hélice ou en spirale dans un état non contraint.

6. Trousse médicale ou ensemble médical (10) selon l'une des revendications précédentes, caractérisé(e) en ce que l'élément coupant (12) est conçu sous la forme d'un ressort de traction, notamment sous la forme d'un ressort de traction hélicoïdal.

7. Trousse médicale ou ensemble médical (10) selon l'une des revendications précédentes, caractérisé(e) en ce que l'élément coupant (12) comporte un métal ou est en un métal qui est de préférence choisi dans le groupe comprenant le fer, l'or, le nickel, le niobium, le platine, l'argent, le tantale, le titane, le vanadium et le zirconium.

8. Trousse médicale ou ensemble médical (10) selon l'une des revendications 1 à 6, caractérisé(e) en ce que l'élément coupant (12) comporte un alliage ou est en un alliage qui est de préférence choisi dans le groupe comprenant les aciers inoxydables tels que les aciers au chrome-nickel, les alliages à base de cobalt tels que les alliages au cobalt-chrome-molybdène (Vitallium) et les alliages de titane comme le nitinol.

9. Trousse médicale ou ensemble médical (10) selon l'une des revendications précédentes, caractérisé(e) en ce que l'élément coupant (12) est conçu en forme de fil, de préférence d'un fil de nitinol.

10. Trousse médicale ou ensemble médical (10) selon l'une des revendications précédentes, caractérisé(e) en ce que l'élément coupant (12) est au moins partiellement revêtu d'un additif, notamment d'un additif visible aux rayons X ou d'un médicament.

11. Trousse médicale ou ensemble médical (10) selon l'une des revendications précédentes, caractérisé(e) en ce que ladite trousse ou ledit ensemble (10) comporte également un fil de guidage (16), notamment un fil de guidage (16) pourvu d'un revêtement hydrophile.

12. Trousse médicale ou ensemble médical (10) selon l'une des revendications précédentes, caractérisé(e) en ce que ladite trousse ou ledit ensemble (10) comporte également un cathéter de guidage, notamment un cathéter de guidage pourvu d'un revêtement hydrophile.

13. Trousse médicale ou ensemble médical (10) selon l'une des revendications précédentes, caractérisé(e) en ce que l'au moins un cathéter à ballonnet (18) est au moins un cathéter à ballonnet (18) revêtu d'un médicament.
